# EUROPEAN PATENT APPLICATION

(11) **EP 4 062 842 A1**
(43) Date of publication of application: **28.09.2022**
(21) Application number: 21164344.0
(22) Date of filing: 23.03.2021
(51) Int. Cl.: A61B 17/16

(54) **RELEASE SYSTEM AND CUTTING PROFILE APPLIED TO DISPOSABLE SELF-LOCKING INTRACRANIAL DRILL BIT**

(71) Applicant: Martos Calvo, Antonio, Diadema (SP) (BR)
(72) Inventor: Martos Calvo, Antonio, Diadema (SP) (BR)
(74) Representative: Carlos Hernando, Borja

(57) **Abstract**

RELEASE SYSTEM AND CUTTING PROFILE APPLIED TO DISPOSABLE SELF-LOCKING INTRACRANIAL DRILL BIT, comprising a disposable cranial drill bit, for single-use, assembled from a coupling mechanism with a internal drill bit and another external one, where an axial load is applied to the cutting edges to transmit the rotary movement to the device, so that, after accessing the cranial bone the mechanism is released and the drill bit ceases its movement, being the coupling mechanism composed by a mechanical arrangement of two sliding helical cams, which interact with a ring, the geometry of which results in coupling, when the drill bit contacts the cranial bone surface, and the drill bit release, once the internal drill bit crossed the bone without affecting the lower cranial layers. The set is encapsulated in a plastic body, which allows the drill bit assembly in a number of craniotomy equipment.

## Description

Cranial surgery procedures, in general, require access to the brain structures. For this purpose, it's necessary to drill holes through the cranial bone so as to access to the neurosurgeon's areas of interest. During the drilling process, the lower layers, such as the meninx or the brain itself, must be preserved.

### State-of-the-art references:

U.S. Patent No. 2.842.131
U.S. Patent No. 4.362.161
U.S. Patent No. 4.319.577
U.S. Patent No. 4.699.550
U.S. Patent No. 4.884.571
   EPO EP 2 022 414 A1
U.S. Patent No. 4.456.010
U.S. Patent No. 4.830.001
U.S. Patent No. 5.380.333

### Other publications:

Acra-cut, Inc. Brochure "Disposable Cranial Perfotators", Acra-cut, Inc., 1989 Main Street, Acton, Massachusetts 01720.

Acra Cut Product Brochure by Acra Cut, Inc. of Acton Massachusetts, 1999.

This patent object is manufactured with materials allowing, if applicable, its sterilization under high temperatures and is used to allow the surgical access to brain tumors, biopsies, hematoma drainage, encephalic cyst aspiration, implantation of interstitial and intracavitary radioactive isotopes, implantation of stimulation electrodes, endoscopy, radiosurgery and other surgical interventions in the cranial region where their determination and precise access is crucial.

It's a double-acting drill bit, with coaxial operation, to be used with a surgical drilling machine.

What is known in the state-of-the art, are drill bits for similar application, but are manufacture under a complex design, being also complex parts, with high operational cost. In case of reuse, the state-of-the-art drill bits are manufactured in such a way that the maintenance and/or cleaning of the set following use is very difficult.

Intracranial surgeries frequently comprise the introduction of an electrode or cannula into the brain so as to reach the affected region and, then, proceed with the appropriate procedure. In this case, we will not mention the second part of the surgery, because it's not important for the proposal claimed at the moment.

To have access to the patient's brain using cannulas and/or electrodes, just the skull drilling by using a drill bit is required to insert the instruments.

There are cranial drillers which allow the drill bit to be rotated only when the drill bit is submitted to an axial load with the desired magnitude. After the load is eliminated or is below a certain pre-determined limit, the drill bit rotation ceases. Such devices, which use a coupling mechanism allowing a driver to be coupled only with sufficient axial load, are disclosed in documents U.S. 2,842,131; 4,362,161; 4,319,577; 4,884,571; and 4,699,550 and EPO EP 2 022 414 A1.

Other previous documents describe reusable drillers which allow the disassembly and assembly for the cleaning and sterilization process. In this case, there's a risk that the drill bit is wrongly assembled and, consequently, comes to fail during the surgery, causing injuries or even the patient death. For this reason, disposable drill bits were developed and started to adopt a plastic body, keeping the mechanism protected and preventing or hindering its disassembly, as disclosed in documents U.S. 4,699,550; 4,456,010; 4,830,001; 5,380,333. Another disadvantage noted in already known cranial drill bits would be the larger number of components or complex geometries, requiring more expensive processes, which affect the final cost of the product. And, finally, the cutting areas noted in other drill bits are not able to reach large thickness bones.

In this proposal, we have a drill bit comprising an "internal cutting shaft", presenting two diameters, being that the largest diameter presents the cutting profile at an end and two integrated cams in the other end, and the smaller diameter serves as a guide to the coupling and releasing mechanism; a secondary drill bit, called "external cutting body" is mounted with a threaded ring, which causes the interaction with the internal cutting shaft cams; a bushing called "dragging trigger", which is responsible for transmitting the rotary movement during the drilling operation; a retaining ring fixed to the smaller diameter of the internal cutting shaft, which maintains the movement of the dragging trigger; a helical spring, assisting with the drill bit release; a body with Hudson-type coupling made of plastic, where the entire set is assembled and sterilized.

The drill bit has a constructive variant, this being of greater length compared to the current drill bits. This resource allows the outside diameter of the external cutting body to be guided through stereotaxic equipment or surgical robots.

The proposed approach allows a single-use process, with improved safety and performance due to reduced moving parts.

This cranial drill bit contemplates a coupling system and cutting profile that are different to the currently existing ones, providing a single and exclusive performance.

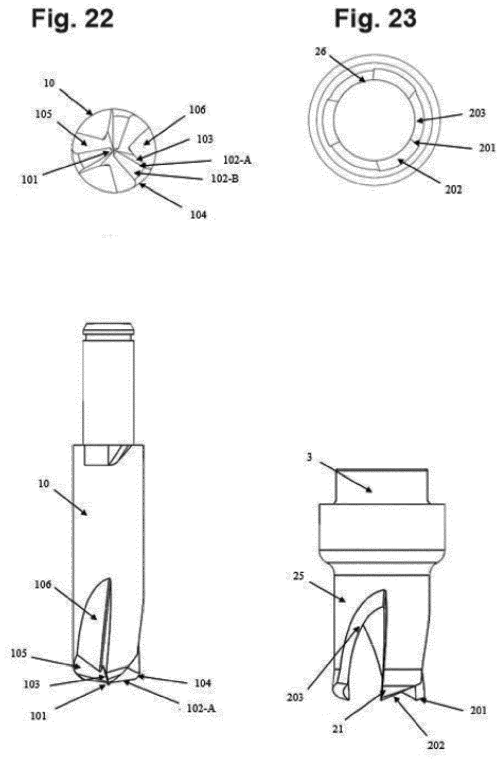

The system will be described according to the attached drawings, provided as follows:
Figures 1 to 9 refer to the general description of the set.
Figures 10 to 22 show the scope of the innovation herein.
Figures 21 to 23 refer to the detailed description of claim 2.
The set of figures is detailed as follows:

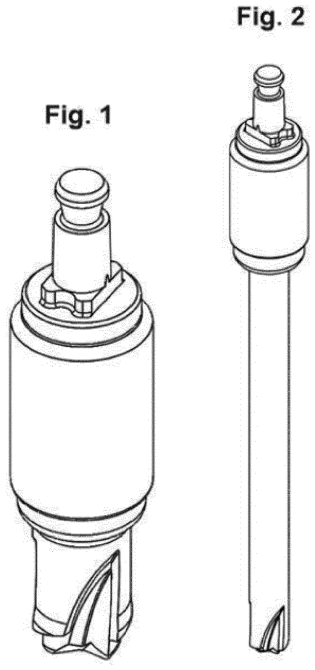

Figure 1 shows the isometric view of the assembled drill bit;
Figure 2 shows a "long version" variant;

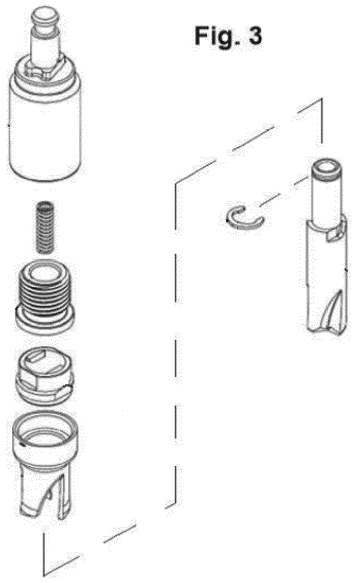

Figure 3 shows the disassembled set in the "short version";

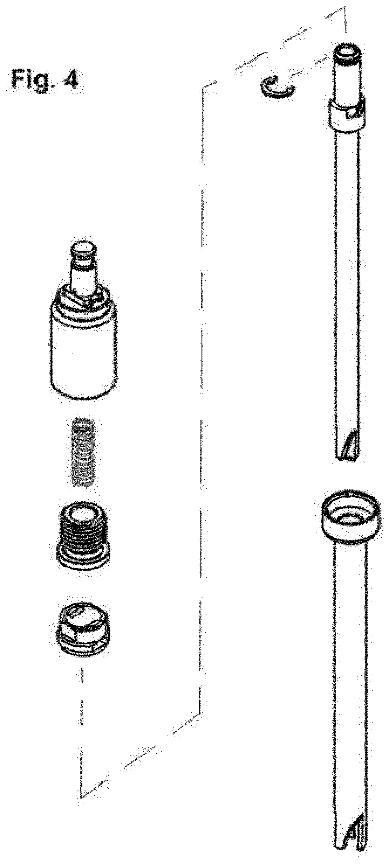

Figure 4 shows the disassembled set in the "long version";

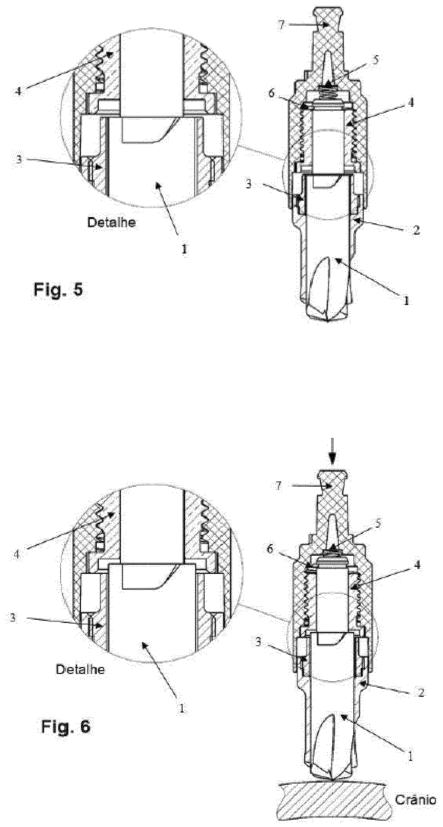

Figure 5 shows the cross-sectional view of the drill bit, in the released condition;
Figure 6 and its enlarged detail show the cross-sectional view of the drill bit, on the cranial bone;

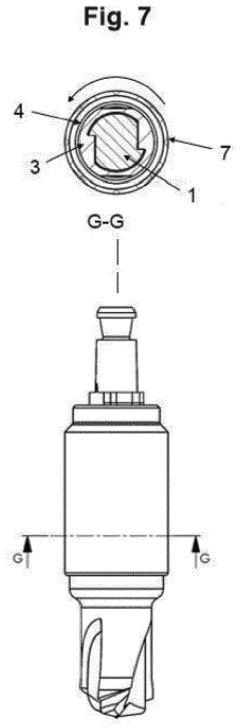

Figure 7 shows a cross-sectional view of the cam section, in the coupled condition during the drilling operation;

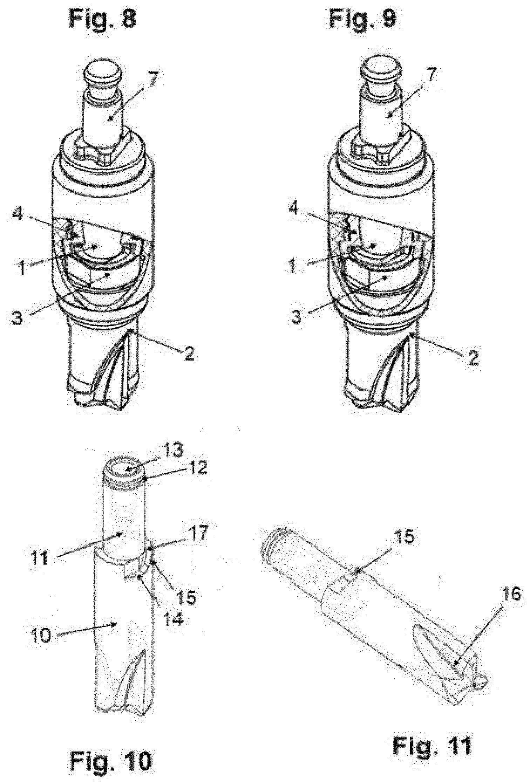

Figures 8 and 9 show the cam interaction with the external body ring in the released and coupled conditions, respectively.
Figures 10 and 11 show details of the internal cutting shaft, comprising a shaft with two diameters, being that the largest diameter presents the cutting profile in one end and two integrated cams in the other one, and the smallest diameter serves as a guide for the dragging trigger holed, so that it couples to the cam and transmits the rotary movement;

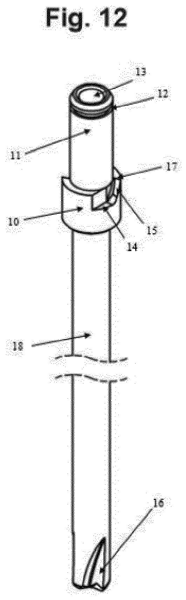

Figure 12 shows the internal cutting shaft in its long construction variant;

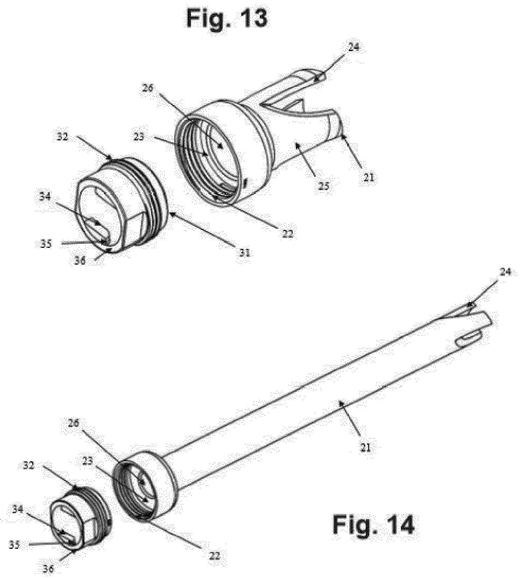

Figure 13 is the exploded view of the external cutting body, where the ring is threaded;
Figure 14 corresponds to the exploded view of the external cutting body, in its constructive variant, with the external body ring;

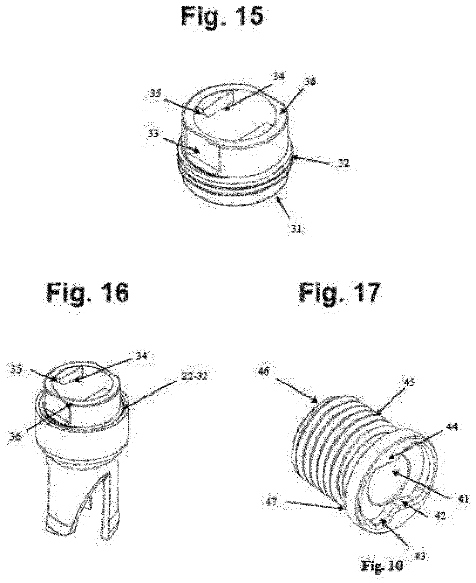

Figure 15 shows the isometric view of the external body ring and the protrusions which interact with the internal cutting shaft cam;
Figure 16 shows the isometric view of the external cutting body ring assemble with the ring, forming a subset which interacts with the internal cutting shaft cams;
Figure 17 shows the isometric view of the dragging trigger;

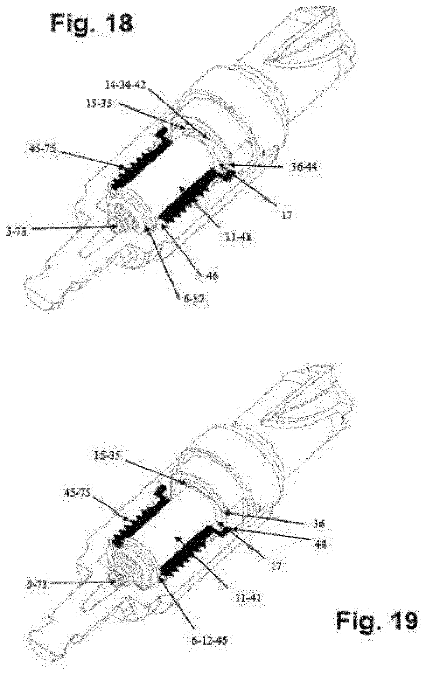

Figure 18 shows a cross-sectional isometric view only of the coupling body and the dragging trigger, representing the set in the coupled condition;
Figure 19 shows a cross-sectional isometric view only of the coupling body and the dragging trigger, representing the set in the released condition;

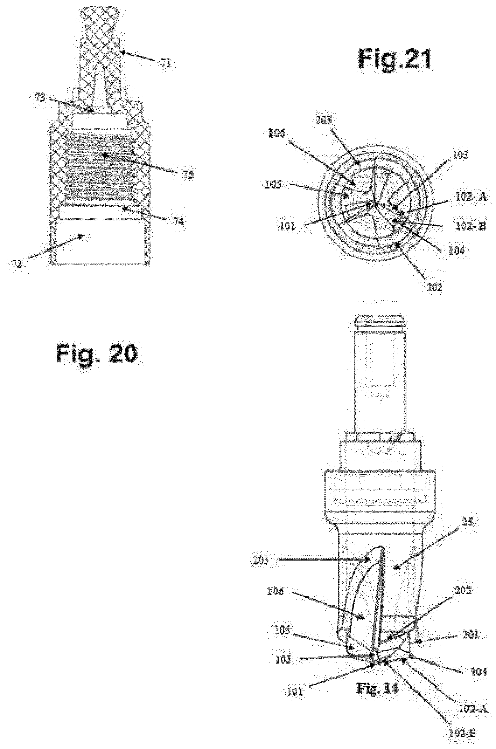

Figure 20 is the cross-sectional view of the "Hudson" standard coupling body.
Figure 21 shows the lower vies and the elevation of the internal cutting shaft and the external cutting body, showing the edge positioning;
The set of figures 22 shows the lower view and the elevation of the internal cutting shaft edge;
The set of figures 23 shows the front view and the elevation of the internal cutting shaft edge.

Figure 1 shows the full assembly of the object of this proposal and Figure 3 shows the exploded view of the parts comprising the referred set, which includes the internal cutting shaft [1], the external cutting body [2], the external body ring [3], the dragging trigger [4], the spring [5], the retention ring [6] and the "Hudson"-type coupling body [7]. Figure 2 shows the full assembly of the long variant and Figure 4 shows the exploded view of the long variant components with the same mechanical arrangement.

The assembly of the set is shown under two operating conditions. Figure 5 shows the cross-sectional view of the drill bit assembly in the rest condition, i.e., in the released condition, where the internal cutting shaft [1] cams remain released from the dragging trigger [4], by means of the spring [5] pressure and the retention ring [6], what limits the movement between the internal cutting shaft [1] and the dragging trigger [4]. The internal cutting shaft [1] and the external cutting body [2] are assemble in such a way that the cutting geometry (G - G) of both remain aligned by the cam faces through the external body ring [3], as shown in Figure 7 section. Such interaction allows that, when connecting the drill bit to a craniotome through the Hudson-type coupling body [7] and, pressing the cranial bone, as shown in Figure 6, the internal cutting shaft [1] cams are coupled to the dragging trigger [4] housing.

The coupling movement is limited between the dragging trigger [4] lower face contact and the upper face of the external body ring [3]. Once the coupling is achieved, the craniotome rotary movement transmission is obtained. By overcoming the bone thickness, the internal cutting shaft [1] is released from the dragging trigger [4], assisted with the spring load [5]. The internal cutting shaft [1] cams also slide, so that the upper face of the largest diameter of the internal cutting shaft [1] coincides with the upper face of the external body ring [3], with a small axial gap of approximately 0.4 mm between the dragging trigger [4] and the external body ring [3]. Thus, the internal cutting shaft [1], the external cutting body [2] and the external body ring [3] remain stuck on the bone, while the other components rotate falsely. This condition is presented in Figure 8 and in Figure 9 and shows the position of the internal cutting shaft [1] cams when the set is pressed and the cams coupled.

The result is a hole with two diameters, being one hole corresponding to the internal cutting shaft [1] diameter [10], which starts between the surface formed by the external cutting body [3], being that in the final portion of such hole a small bone disk is formed in order to help to protect the dura mater (known as "bone pad"); and a second lowered hole between the bone surface, until a distance which may range between 1 to 3 mm before the end of the first hole, corresponding to the external cutting body [2] diameter [21].

Concerning Figure 1, below we depict the individual parts of the drill bit which will be described with more details. The internal cutting shaft [1], shown in Figures 10 and 11, comprises a shaft with two diameters [10] and [11], being that the largest diameter [10] presents the cutting profile [16] in one end, which will be described later on, and two cams opposed to each other, in the other end. Diameter [10] has a constant diameter or approximately 14 mm. Figure 11 present the construction variant of the internal cutting shaft, which also presents a diameter [10] of approximately 14 mm, but with a length of approximately 9 mm from the upper face [17]. As shown in Figure 12, from the lower face, the internal cutting shaft has a second diameter [18], which may range between 5 mm and 8 mm, with lengths between 25 mm and 130 mm, with the same cutting profile [16] shown in Figure 11.

Each of the cams comprise two faces, being that one flat face [14] contacts the external body ring face [34], shown in Figures 13, 14, 15 and 16. Such contact ensures the alignment of the cutting geometries [16] and [24], shown in Figures 8 and 9, allowing the rotary movement dragging between the internal cutting shaft [1] and the external cutting body [2]. One portion in faces [14] and [15] is exposed in approximately 1.2 mm and contact the dragging trigger face [42], shown in Figures 17, 18 and 19, being responsible for transmitting the craniotome rotary movement to the internal cutting shaft and to the external cutting body [2]. The other came face [15] starts as a flat base and follows from the center with a helical upward slope of approximately 36 mm of pace to the face [17].

Said surface [15] contacts the external body ring face [35], shown in Figures 13, 14, 15 and 16 during drilling. When the bone thickness is overcame, the dragging occurs between them allowing the release, as shown in Figures 13, 14, 15 and 16. The largest diameter [10] serves as a guide for the part [2] hole [26], shown in Figures 13 and 14. The smallest diameter [11] serves as a guide for the dragging trigger hole [41], shown in Figure 17. The upper end of the part [1] is provided with a groove [12], where the retention ring [6] is assembled. Such ring limits the movement between the dragging trigger [4] and the external body ring [3] during the drill bit coupling and release, shown in Figures 18 and 19 and, more specifically, it limits the groove [12] of Figures 10, 11 and 12, the external body ring face [36] of Figure 8 and the dragging trigger faces [44] and [46] in Figure 17.

The upper face of the internal cutting shaft [1] is provided with a hole [13] which serves as a housing for the spring [5].

The external cutting body [2] is assembled with the external body ring [3], forming a subset, as shown in Figures 13, 15 and 16. The external cutting body has a hole [26], where the internal cutting shaft [1] is assembled by means of a thread [22] in the upper end where the external body ring thread [32] is assembled and a flat seating surface [23] of the external body ring in the face [31]. The ring can be fixed by means of a spanner in the flat faces [33]. The other end is provided with the cutting profile [24] and a diameter [21] measuring between 9 and 14 mm and length of approximately 20 mm, which includes a recess [25], the role of which is to reduce the lateral friction during the drilling operation of thicker bones.

The long construction variant shown in Figure 14 has a diameter [21] measuring between 9 mm and 12 mm with a length of approximately 120 mm. Another difference in the long variant shown in Figure 14, compared to Figure 13, is that the recess [25] is not present, because the diameter [21] will serve as a guide to be used in stereotaxic instruments or surgical robots.

As previously shown the dragging profile of the cams is formed by protrusions 34 /35 (Figure 15) and interact with cams [14] and [15], shown in Figures 10, 11 and 12 and which also interact with protrusion [42] (figure 17) in the drill bit coupling and releasing mechanism, shown in Figures 18 and 19.

The dragging trigger [4] is a metal sleeve, made of bronze, formed by hole [41] (Figure 17) with sliding adjustment between the latest and diameter [11] of the internal cutting shaft [1] (Figures 10, 11 and 12). It is provided with a housing [42] and [43] where internal cutting shaft [1] cam faces [14] contact to couple and release the set during the drilling operation.

The lower face [44] remains in contact with the upper face [36] of the external body ring, when the set is coupled during the drilling operation. In the condition that the drill bit is released, face [46] remains in contact with the retention ring [6]. It is provided with a thread [45] which is assembled in thread [74] in the Hudson-type coupling body [7] (Figs. 17 and 20).

The entire drill bit coupling and releasing mechanism of this proposal is encapsulated in the plastic Hudson-type coupling body [7]. It has a body [71], where the coupling device is mounted in the craniotome, being that the coupling device model may range according to the craniotome by other manufacturers, such as the Smith-type coupling device, for example. Diameter [72] serves to cover the external body ring [3], the upper part of the external cutting body [2] and the two cams formed by [14], [15] and [17] (Figures 11 and 12). Spring [5], mounted in part [1] housing [13] (Figure 1), is also supported on the coupling body housing [73] (Figure 20), so that it may have enough load to assist in releasing the drill bit cams, shown in Figures 18 and 19.

As previously mentioned, thread [75] is assembled with the dragging trigger [4] thread [45] and its seating in Hudson-type coupling device [7] is performed by the contact between the faces [47 and 74] (Figures 17 and 20).

As previously described, the internal cutting shaft [1], shown in Figures 10 and 11, comprises a shaft provided with two diameters [10 and 11], being that the largest diameter [10] is provided with the cutting profile [16] in one end, as shown in Figures 11 and 13 and the external cutting body [2] cutting profile [24].

It has also been described that the interaction between the external body ring [3] faces [34 and 35] ensures the alignment of the cutting geometries [16 and 24], shown in Figures 15 and 16 and allows the rotary movement dragging between the internal cutting shaft [1] and the external cutting body [2].

The set in Figure 21 shows the assembly in the coupled condition between the internal cutting shaft [1], the external cutting body [2] and the external body ring [3], where the alignment of the departure angles formed by [106] and [203] is identified. There's also a distance between the point angle [102] edge and the point angle [201], which may range between approximately 1 and 3 mm. The desired alignment and positioning are obtained by the fact that the sharpening process is conducted with the set assembled. Figures 15, 16, 17 and 18 show the lower and front views of the internal cutting shaft [1] and the external cutting body [2].

Starting with the internal cutting shaft [1], this is formed by the body [10], the cut of which results in the smallest diameter of the hole. As shown in the set of Figures 22 and 23, the internal cutting shaft comprises a tip 101 of 0.4 mm, which allows the drill bit to remain positioned to perform the hole. The cutting geometry id formed by the point angle [102-A] of approximately 7° from the base, between the point edge [101] to the face [10], with a clearance angle [102-B] of approximately 20° from the base. From the point [101], we have a facet [103], the angle of which is negative in approximately 15° with respect to the drill bit shaft. The edge formed between the intersection of angles [102-A], [102-B] and [103] has a rounding [104], so as to soften the internal cutting shaft cut [1] (set of Figures 21) and the formation of the bone disc, known as "bone pad". There's also a second faceted corner [105], perpendicular to the facet [103] and with an angle of approximately 40°from the shaft, which decreases the clearance angle [102-B] contact area, favoring the release of chips (see elevation in Figure 22). Concerning the release of chips, there's also the geometry of the departure angle [106] and [206], with a slope of approximately 6° with respect to the internal cutting shaft [1] center line (Figures 21, 22 and 23).

As shown in Figures 21 and 22, the departure angle faces [106] and [203] profile form an angle of approximately 75°. This profile describes an arc trajectory [16], shown in Figures 10, 11 and 12, starting from the internal cutting shaft [1] end [101] and ending in the external cutting body [2] recess face [25], in a vertical distance of approximately 20 mm (Figures 21, 22 and 23).

As shown in Figure 21, the sharp faces are positioned at 120° between them, forming the tip profile [101] and a minimum frame diameter of approximately 4 mm in the internal cutting shaft [1]. The result of this positioning is a higher volume to release the chips, allowing to reach bones with higher thicknesses.

The external cutting body [2] is formed by two diameters [21 and 25] (Figure 23). Diameter [21], the cut of which results in the lowered hole diameter, has a recess [25] (Figure 23), the role of which is to reduce the lateral friction during the drilling of thicker bones. It's important to emphasize that, as previously described and shown in Figure 14, the long construction variant is not provided with the recess [25], because the diameter [21] may be coupled to stereotaxy-driven instruments or in surgical robots, guaranteeing increased precision in the drilling trajectory. As shown in Figure 23, the geometry is formed by the point angle [201] of approximately 15° from the base, between the inner diameter [26] and the outer diameter [21], having a clearance angle [202] of approximately 20°, in parallel with the clearance angle [102-B] of the internal cutting shaft [1] (Figure 21).

As previously described, the departure angle [203] is formed in combination with departure angle [106] (Figure 21) so that, during the drilling operation, the surfaces formed by [106] in the internal cutting shaft [1] and [203] in the external cutting body [2] coincide. The characteristics of the described sharpening, cutting angle, point angles and clearance angles geometries, also apply for the long construction variant.

## Claims

1. RELEASE SYSTEM AND CUTTING PROFILE APPLIED TO DISPOSABLE SELF-LOCKING INTRACRANIAL DRILL BIT, **characterized by** the fact that the dragging geometry through opposed cams is formed by the interaction between the internal cutting shaft [1] flat face [14] and helical slope [15], being the dragging profile formed by [34 and 35] of the external body ring [3], as well as also the interaction between the dragging trigger [4] housing [42 and 43], where the internal cutting shaft [1] of the cam faces [14] contact to couple and release the set.

2. RELEASE SYSTEM AND CUTTING PROFILE APPLIED TO DISPOSABLE SELF-LOCKING INTRACRANIAL DRILL BIT, according to claim 1, **characterized by** the fact that the combination between the internal cutting shaft [1] and the external cutting body [2] departure angle profiles [106 and 203], in combination with facet [105], applied to the clearance angle in the internal cutting shaft [1], in addition to the external cutting body [2] recess [25], are intended for the release of chips.
